# EUROPEAN PATENT SPECIFICATION

(11) **EP 1 104 675 B1**
(45) Date of publication and mention of the grant of the patent: **16.08.2006**
(21) Application number: 00941043.2
(22) Date of filing: 15.06.2000
(51) Int. Cl.: A61K 33/18, A61K 38/16, A23L 1/305, A23L 2/66, A61P 5/14, A61P 5/16

(54) **IODINATED PROTEINS FOR PREVENTING IODINE-DEFICIT CONDITIONS**
JODIERTE PROTEINE ZUR VORBEUGUNG VON JOD-MANGEL ZUSTÄNDEN
PROTEINES IODEES POUR PREVENIR DES ETATS DE DEFICIT EN IODE

(30) Priority: 18.06.1999 RU 99112675
(43) Date of publication of application: 06.06.2001
(73) Proprietor: Obschestvo C Ogranichennoi Otvetstvennostiju Medbiofarm, Kaluzhskaya obl., 249020 (RU)
(72) Inventor: TSYB, Anatoly Fedorovich, Kaluzhskaya obl., 249020 (RU); ROZIEV, Rakhimdzhan Akhmetdzhanovich, Kaluzhskaya obl., 249020 (RU); SKVORTSOV, Valery Grigorievich, Kaluzhskaya obl., 249020 (RU); KLEPOV, Alexandr Nikolaevich, Kaluzhskaya obl., 249020 (RU); SKOBELEV, Igor Valentinovich, Kaluzhskaya obl., 249050 (RU); US, Pavel Petrovich, Kaluzhskaya obl., 249020 (RU); KUZIN, Viktor Vasilievich, Kaluzshkaya obl., 249020 (RU); GONCHAROVA, Anna Yakovlevna, Kaluzhskaya obl., 249020 (RU); BOZADZHIEV, Leonid Lukiyanovich, Kaluzhskaya obl., 249020 (RU); GRIGORIEV, Alexandr Nikolaevich, Kaluzhskaya obl., 249020 (RU)
(74) Representative: von Füner, Nicolai
(86) International application number: PCT/RU2000/000230
(87) International publication number: WO 2000/078321

(56) References cited:
- EP-A1- 0 401 109
- WO-A1-00/03610
- FR-M- 3 869
- RU-C1- 2 110 265
- RU-C1- 2 134 520
- SU-A- 560 581
- US-A- 3 153 615
- US-A- 4 631 270
- US-A- 5 059 679
- DATABASE CA [Online] CHEMICAL ABSTRACTS SERVICE, COLUMBUS, OHIO, US; GUYANG, AN ET AL: "Experimental study on casein prevention of iodine - deficient goiter in rats" retrieved from STN Database accession no. 119:71459 HCA XP002241867 & ZHONGHUA NEIFENMI DAIXIE ZAZHI (1992), 8(3), 159-60 ,
- MARIAN ELLIOTT KOSHLAND ET AL.: 'Modification of amino acid residues in anti-p-azabenzenearsonic acid antibody during extensive iodination' THE JOURNAL OF BIOLOGICAL CHEMISTRY vol. 238, no. 4, April 1963, pages 1343 - 1348

## Description

The present invention relates to the food industry and can be used in medicine and pharmacology and, in particular, in the production of bread, confectionary and pasta, milk products, children's food, fruit juice and other beverages such as kvass and beer, for manufacturing vitamin and mineral complexes which are to be used for preventing iodine deficit.

The problem of iodine deficit is a global problem. The populations of many countries have a lack of iodine what leads to diseases of thyroid gland and metabolic disturbances which are capable to go over into oncological diseases. Another side of iodine deficit is low growth and mental lethargy.

Known is the use of iodized table salt in order to compensate an iodine deficiency occurring in an organism. The iodized salt represents a mechanical mixture of common salt (NaCl) and the inorganic compounds potassium iodide (KI) or potassium iodate (KIO₃) /1/. As modern researches have shown, there are some disadvantages in applying iodized salt. The iodine content in iodized salt does not allow to administer precise dosages thereof. To the first, this is connected to the high volatility of iodine. During storage and transportation of the product the content of iodine therein decreases considerably. Preparations of potassium iodide are very unstable to the effect of light and moisture. Over a period of 3 months the content of inorganic iodine in an iodized salt decreases to 50%. Potassium iodate is more stable. However, it is unstable, too, at the presence of impurities of other inorganic salts, at the presence of moisture or temperature drop, at the effect of weak acids or alkaline conditions. The instability of these compounds will be increased during the heat treatment of food such as bread baking which is characterized by the presence of both acidic and alkaline conditions in the time of preparing and fermentation of the dough and is also characterized by the effect of high temperatures of up to 220°C in the oven. Secondly, as researches of world-wide organisations that are considering the question of iodine deficit have shown, the imperfections of the present methods of mixing salt with iodine preparations result in that the potassium iodide (iodate) level in salt varies from 0 to up to 600 parts per million (on an average data from 24 to 148 parts per million) /2/. In Russia there is used a iodized salt having a iodine level of 40±15 µg per gram of product. At a daily consumption of 10 to 15 g of salt, 375 to 825 µg of iodine can reach the thyroid gland per day, what exceeds the physiological standard by 2.5 to 5.5 times. Such high iodine concentrations in the thyroid gland may cause rather undesirable results: retardation in the synthesis of thyroid hormones in the thyroid gland, disturbance of metabolic regulation, development of autoimmune thyroiditis and other diseases of the thyroid gland. The data of foreign researches /3,4/ obtained on the basis of the analysis of a representative selection indicate a sharp increase in the occurrence of thyroid gland diseases as a result of utilizing inorganic iodine preparations at an elevated dosage. This disadvantage is caused by the fact that the organism actually does not participate in the regulation of the iodine intake from inorganic compounds into the thyroid gland, because iodine, while by-passing the liver and gastro-intestinal tract, goes through the blood entirely into the thyroid gland and may inhibit the functions thereof when the intake is elevated. There are also other negative effects when iodized salt is utilized which, e.g., result in a worsening of organoleptic properties of the food such as the odour, taste. Further, the technological ineffectiveness of the production of iodized salt is to be noted which is due to the fact that it requires a precise and expensive equipment for the mixing of microquantities of iodine preparations with macroquantities of salt.

Also known is the utilization of a starch-iodine complex for the treatment and prevention of diseases caused by lack of iodine /5/. The starch-iodine complex as an organic compound exhibits smoother and more natural effects because the gastro-intestinal tract is involved in the digestion thereof. The use of said preparation increases the accuracy of the iodine dosage administered to an organism but, however, is also insufficient since in starch the ratio of amylase as the carrier of iodine to amylopectin may vary and depends on the variety, quality, time of harvesting and origin of the plants from which said starch has been prepared. Usually, the amount of iodine in a known complex as such will be determined by the amount of iodine that appears in said complex but not by the amount of iodine that could be bound to the amylose; and therefore, the composition comprises iodine in an inorganic form as a so-called inclusion compound. Thus, as well as in the case of employing inorganic iodine, the utilization of starch-iodine complexes does not allow an exact dosage and does also not allow for an individual regulation of the iodine metabolism due to the mode of iodine release from starch in the organism. A further disadvantage is that starch-iodine complexes can be administered only in dry form like capsules or tablets and cannot be applied in form of a food additive as they begin to decompose already at 40°C.

Known are foodstuffs that include iodine-containing food additives. Such products are bread, cakes and pastries, milk, oils /6,7/. As said iodine-containing food additives in said foodstuffs, there have been used inorganic iodine compounds, laminaria (Crambe), yeast cultures which have been cultivated in iodinated water.

The disadvantage of these known products is that they have a distinct disagreeable aftertaste and odour as they include inorganic iodine compounds which may disintegrate and release free iodine upon the effect of light. It has become known that also Crambe possesses a rather big reserve of inorganic iodine compounds. The level of organic and inorganic iodine compounds in Crambe varies considerably in dependence of the place of growing, growing conditions, processing and transportation techniques.

All these known products do not allow to individually regulate the iodine exchange in an organism and, moreover, iodine volatilizes therefrom during the processing and storage of the products.

The problem to be solved by the authors was to provide an agent that would allow to supply the organism with the deficient iodine, and would also enable the individual regulation of iodine exchange. At the same time, such a composition must keep its properties constant over a prolonged period of time, must not have a taste and odour, the production thereof must be technologically effective, and its handling must be easy.

To solve this problem, it is proposed to use an iodinated protein and/or a low-molecular component thereof, namely a polypeptide or peptide the structure of which includes at least one amino acid of the following group: tyrosine, phenylalanine, histidine, tryptophane, for the preparation of an agent contained in a vitamin complex or in a mineral complex for preventing iodine deficit conditions.

In an embodiment the agent may comprise iodinated casein or iodinated lactoglobulin.

The agent can be included in the formulation of a foodstuff or a beverage,

The use of the iodinated nutrient protein allows to compensate iodine deficit because in the case of consumption of said agent the following mechanism of iodine ingestion takes place: gastro-intestinal tract - liver - thyroid gland. Due to this mechanism the organism assimilates the required dosage of iodine and excretes the redundant iodine out of the organism without causing any harm. The preparation is completely soluble in water and does thereby not lose its properties over several days. When it is employed in a foodstuff the preparation does not have worse organoleptic properties, neither odour nor taste nor color. The preparation maintains its properties stable over a prolonged period of time. For example, a iodinated casein powder can be stored up to 12 months in a paper package in a dry, dark place at a temperature of not more than 25°C. Iodinated proteins are stable during the baking of bread and preserve for a long time constant properties in finished products like cakes and pastries, children's food, confectionary and milk products. The period over which the iodinated proteins stably exist in said products is comparable to the period of storage stability of said products or does even exceed it.

The invention is based on a mechanism, which has been disclosed by the authors for the first time, regarding the regulation of iodine metabolism in the organism of iodine-containing proteins and/or low-molecular components thereof that come from outside.

The preparation functions as follows. In the gastro-intestinal tract the iodine-containing preparation disintegrates up to the iodine-containing amino acids. The entering of these amino acids from the gastro-intestinal tract into the liver is accompanied by the splitting off of iodine under the effect of an enzyme (deiodinase). The activity of this enzyme depends directly on the degree of iodine deficit and the functional status of the thyroid gland. The excessive amount of iodinated amino acids, most of all iodotyrosines, will be transformed into glycuronides and excreted by the organism. Therefore, an overdosage of said agent is in principle impossible.

The authors have carried out experimental tests on iodinated casein which support the functional suitability and safety of its use. The uptake of iodinated casein was in good correspondence to the status of the organism, as it has been more gathered in at iodine lack, and to a lesser degree in case of sufficient iodine supply to the organism. Both the acute and chronic toxicity of iodinated casein were studied. As a result thereof it has been established that an excess of the recommended single dose by 1000 times and an excess of the daily dose by 100 times over 30 days does not cause any visible changes in the organisms of the tested animals. An assessment of the allergic properties of iodocasein was carried out and has shown that iodocasein does not induce allergic reactions. Analogous data was obtained for iodinated lactoglobulin.

The substance can be produced as follows. Proteins, e.g., from milk or milk protein hydrolysate are dissolved in a buffer to obtain a suitable concentration thereof. For the iodination can be used either dry proteins, or hydrolysate, or a finished protein solution such as milk or a protein hydrolysate solution. The obtained solution is iodinated by means of iodine chloride, or chloramine T (or chloramine B), or by means of enzymes, at a stoichiometric ratio, or at an excess of the iodination agent for complete utilization of the protein, or at a deficit of iodination agent for complete utilization of iodine. In case an excess of iodination agent is used, the solution is purified from free iodine by means of a chemical reducing agent such as Na₂SO₃, NaS₂O₃ etc. The iodinated proteins precipitate from the solution upon reduction of the acidity up to a pH of 3 to 4. The protein precipitate is removed from the solution by methods like filtration or centrifugation. The isolated, washed and purified iodinated proteins are dried up to a moisture content of at most 5 % by lyophilization, spray drying or any other method.

The agent is to be used as follows. The powder of iodinated protein is dissolved in water in a ratio of 1 g of powder per 100 mL water. The obtained solution is added to an aqueous phase which shall be used in the preparation of a foodstuff, beverage, vitamin or mineralized complex. The preparation of said products follows the iodine ingestion levels recommended by the WHO, which differ in dependence of the age-group of the consumers.

For example, to prepare 1 metric ton of bread 5 g iodocasein were dissolved in 500 mL water. The obtained solution is added to an aqueous phase that is to be used for the preparation of the dough. The further process for the manufacture of the bread is carried out by means of known techniques.

It is recommended to use iodocasein as additive in protein- containing beverages, e.g. kvass, beer, milk, kefir etc. For this purpose, the dry iodocasein powder is added directly into the brewing wort during the preparation process of the beverage in an amount of 5 mg per 1 L of beer or kvass, or in an amount of 2 mg per 1 L of milk products (milk, kefir etc.).

In milk preparations for children's food of the 'Malysh' type, there is added iodocasein in an amount of 6 mg per 1 kg of the mixture. In calcium glucanate tablets the dosage of iodocasein is 1 mg per tablet at a daily administration of 2 to 3 tablets.

The present invention allows a more effective solution of the problem of iodine deficit.

### REFERENCES

1 Monitoring Universal Salt Iodization Programs, Published by PAMM/ICCIDD/MI, 1995
2 Conference to ensure the guarantee of the quality of salt iodization programs, October 1996, (in Russian)
3 The incidence of hyperthyroidism in Austria from 1987 to 1995 before and after increase in salt iodization, Mostbeck A., Jur. J. Nucl. Med. 25, 367-374 (1998)
4 Target Organ Defects in Thyroid Autoimmune Disease, Roy S. Sundick, Immunol. Res., 1989; 8: 39-60
5 Patent RU 2,110,265, IPC A61K 33/18, publ. 10.05.1998, Bul. No. 13
6 Collection of prescriptions and technological instructions for the production of dietetic and prophylactic types of baked goods, National Scientific Research Institute of the Bread Baking Industry (NSRIBBI), Moscow, Foodstuffs, 1997, (in Russian)
7 The iodine-enriched food additive 'Amiton', Technological Regulations (TU), Technological Instructions (TI), Prescription (RZ) 9110-273-05747152-98 (NSRIBBI)

## Claims

1. Use of an iodinated protein and/or an iodinated low-molecular component thereof, i.e. an iodinated polypeptide or peptide the structure of which includes at least one amino acid of the following group: tyrosine, phenylalanine, histidine, tryptophane, for the preparation of an agent contained in a vitamin complex or in a mineral complex, for preventing iodine deficit conditions.

2. The use according to claim 1, **characterized in that** said iodinated protein is iodinated casein.

3. The use according to claim 1, **characterized in that** said iodinated protein is iodinated lactoglobulin.

4. The use according to claim 1, **characterized in that** the agent is contained in a foodstuff.

5. The use according to claim 1, **characterized in that** the agent is contained in a beverage.

## Patentansprüche

1. Verwendung eines iodierten Proteins und/oder einer iodierten niedermolekularen Komponente davon, und zwar eines iodierten Polypeptids oder Peptids, dessen Struktur wenigstens eine Aminosäure der Gruppe Tyrosin, Phenylalanin, Histidin und Tryptophan umfasst, zur Herstellung eines in einem Vitaminkomplex oder in einem Mineralkomplex enthaltenen Mittels zur Verhinderung von Iodmangel.

2. Verwendung nach Anspruch 1, **dadurch gekennzeichnet, dass** das iodierte Protein iodiertes Casein ist.

3. Verwendung nach Anspruch 1, **dadurch gekennzeichnet, dass** das iodierte Protein iodiertes Lactoglobulin ist.

4. Verwendung nach Anspruch 1, **dadurch gekennzeichnet, dass** das Mittel in einem Nahrungsmittel enthalten ist.

5. Verwendung nach Anspruch 1, **dadurch gekennzeichnet, dass** das Mittel in einem Getränk enthalten ist.

## Revendications

1. Utilisation d'une protéine iodée et/ou d'un composant iodé de faible poids moléculaire de celle-ci, c'est-à-dire un polypeptide ou peptide iodé dont la structure comprend au moins un acide aminé du groupe suivant : tyrosine, phénylalanine, histidine, tryptophane, pour la préparation d'un agent contenu dans un complexe vitaminé ou dans un complexe minéral, afin d'empêcher des conditions de déficit en iode.

2. Utilisation selon la revendication 1, **caractérisée en ce que** ladite protéine iodée est la caséine iodée.

3. Utilisation selon la revendication 1, **caractérisée en ce que** ladite protéine iodée est la lactoglobuline iodée.

4. Utilisation selon la revendication 1, **caractérisée en ce que** l'agent est contenu dans un aliment.

5. Utilisation selon la revendication 1, **caractérisée en ce que** l'agent est contenu dans une boisson.
